(19) Europäisches Patentamt
European Patent Office
Office européen des brevets

(11) Publication number: 0 257 716 A2

(12) # EUROPEAN PATENT APPLICATION

(21) Application number: 87201590.4

(22) Date of filing: 21.08.87

(51) Int. Cl.4: C12P 1/00 , C12P 7/40 , C12P 41/00

(30) Priority: 22.08.86 US 898972

(43) Date of publication of application: 02.03.88 Bulletin 88/09

(84) Designated Contracting States: BE DE FR GB

(71) Applicant: STAUFFER CHEMICAL COMPANY

Westport Connecticut 06880(US)

(72) Inventor: Dahod, Samun Kalim
1673 Saddle Hill Road
Green Oaks, IL 60048(US)

(74) Representative: Smulders, Theodorus A.H.J. et al
Vereenigde Octrooibureaux Nieuwe Parklaan 107
NL-2587 BP 's-Gravenhage(NL)

(54) **Improved process for conducting lipase enzyme hydrolysis.**

(57) Lipase hydrolysis of an ester group of a substrate can be conducted in the absence of large quantities of water and excess enzyme without extensive inhibition of enzyme activity due to water-soluble product alcohol. A lipase reaction can be efficiently conducted by dispersing a water-immiscible organic substrate phase in an aqueous phase, washing the reaction continuously while the reaction mixture is maintained in the dispersed state, and continuously removing an amount of aqueous phase substantially equivalent to the washing solution added. The washing solution can be an aqueous neutralizing solution. By this process, water-soluble product alcohol can be removed from the reaction mixture without removal of substantial quantities of water-soluble enzyme.

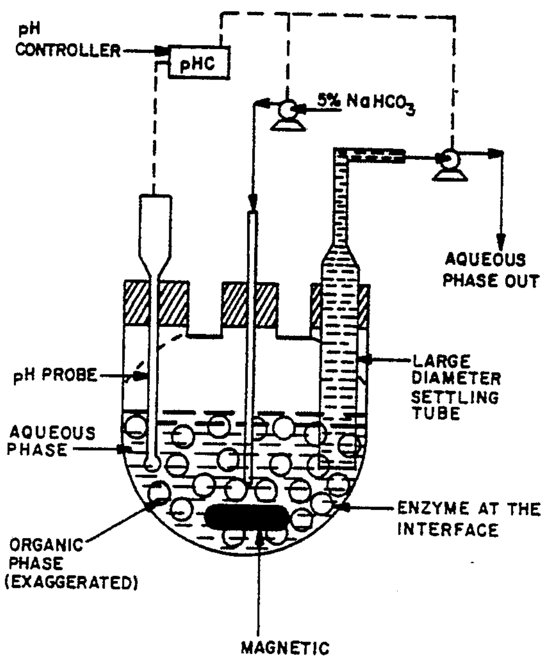


FIG.1

EP 0 257 716 A2

# IMPROVED PROCESS FOR CONDUCTING LIPASE ENZYME HYDROLYSIS

The present invention relates to an improved process for conducting lipase enzyme hydrolysis reactions.

## BACKGROUND OF THE INVENTION

In addition to using lipase esterase enzyme to hydrolyze ester groups from fats, certain lipase enzymes can also be used to resolve stereoisomers by the stereospecific hydrolysis of one of the isomers. For example, DL-octyl-2-chloropropionate can be resolved by hydrolyzing the racemate with a stereospecific lipase from Candida cylindracea, the D isomer acid being formed along with an equal amount of product alcohol. The so formed acid is readily separable from the substrate ester (Lipase-Catalyzed Production of Optically Active Acids Via Asymmetric Hydrolysis of Esters, Applied Biochemistry & Biotechnology, Vol. 9, No. 3, pp. 255-260 - 1984).

In many lipase catalyzed reactions, the water-soluble alcohol resulting from the reaction inhibits the activity of the enzyme. For this reason, such lipase hydrolysis reactions have to be run in a batch process with sufficient water to dilute the alcohol formed as the product of the reaction. The ratio of water to substrate depends on the molecular weight of the ester. Typically, for methyl 2-chloropropionate the ratio is about 4 to one. The effect of the progressive increase in the concentration of alcohol is the reduction of enzyme activity. For an effective process, excess water and excess lipase are required to offset any loss in enzyme activity due to product alcohol. The requirement for large quantities of water limits the amount of reactant that can be hydrolyzed per reaction batch thereby limiting yield. The requirement of a dilute batch reaction, excess water and excess enzyme along with low yields complicates the economics of the process.

In addition, the stereoselectivity of an enzyme can be affected by the solubility of the substrate. As mentioned before, the octyl ester of 2-chloropropionic acid which is essentially water insoluble can be resolved enantiomerically with a lipase enzyme from Candida cylindracea. The same article states that the methyl ester which is more water soluble cannot be so resolved.

While enzyme reactions are favored in an aqueous system, water-immiscible substrates impose a limitation on the ability of enzymes to catalyze a reaction. Water-miscible solvents for the substrates, such as alcohols, are undesirable as they have a denaturing effect on the enzymes. In U.S. Patent 3,926,726, a two-phase solvent system is used, an aqueous phase for the enzymatic reaction and a water-immiscible organic solvent phase which acts as a reservoir for the substrate and for the continuous extraction of the reaction products from the aqueous phase to avoid inhibition of the enzyme activity.

While the use of a two-phase aqueous/water-immiscible solvent system is effective in conducting enzyme reactions on water-immiscible substrates, enzyme inactivation and inhibition due to water-soluble reaction products remains a problem since the enzymatic reaction is conducted in the aqueous phase. Removal of products along with the aqueous phase at the expense of enzymes or enzyme activity can be economically disadvantageous due to the high cost of the enzyme.

## SUMMARY OF THE INVENTION

It has been found that lipase hydrolysis of an ester group of a substrate can be conducted in the absence of large quantities of water and excess lipase at good reaction rates without extensive inhibition of the enzyme activity due to the alcohol formed as a result of the reaction.

According to the process of the invention, a lipase enzymatic reaction can be conducted by sufficiently dispersing an organic substrate-containing phase in an aqueous phase under conditions such that the organic phase is not miscible with the aqueous phase in the presence of a water-soluble non-immobilized lipase enzyme so that substantially all of the enzyme is bound to the substrate at the interface, and continuously adding an aqueous washing media and continuously removing a substantially equal volume of aqueous phase containing the alcohol or other enzyme inhibitors or products but not a substantial amount of enzyme as the reaction proceeds. By conducting the lipase enzymatic reaction under these conditions, portions of the aqueous phase containing water-soluble alcohol product can be continuously removed from the reaction mixture without substantially decreasing the enzyme concentration in the reaction mixture.

The invention finds particular advantage in preparing alkyl esters of L-2-halopropionic acid known to be useful in preparing isomeric phenoxy propionate herbicides.

BRIEF DESCRIPTION OF THE DRAWING

Figure 1 is a representation of an apparatus for use in the process of the invention.

DETAILED DESCRIPTION OF THE INVENTION

The process of the invention comprises enzymatically reacting a substrate which is not miscible with an aqueous phase with a non-immobilized lipase enzyme under reaction conditions adopted to bind the enzyme to the substrate at the interface between the aqueous phase and the water-immiscible organic phase which allows separation of the aqueous phase from the organic phase without substantial loss of the water-soluble lipase enzyme.

By "substrate" as used herein is intended to mean the substance acted upon by the lipase enzyme. A substance which cannot be acted upon by a specific enzyme is not a substrate for that enzyme.

By the term "substantially all the enzyme is bound" is intended to mean that at least 80% and preferably at least 95% of the lipase enzyme is bound in some manner to the water-immiscible organic substrate phase when equilibrated.

The substrates are esters wherein the ester group is hydrolyzable under the catalytic influence of a lipase enzyme. The substrate must also be capable of forming a dispersed organic phase in an aqueous medium, i.e., a liquid at the temperature of reaction or soluble in an organic solvent which is compatible with the enzyme, both very substantially water immiscible. Suitable substrates include fatty acid esters wherein the acid moiety has from about 1 to about 22 carbon atoms and the alcohol moiety has from about 1 to about 4 carbon atoms. The alcohol from the alcohol moiety is water soluble so that the product alcohol can be separated with the aqueous phase. The fatty acid esters can be saturated or unsaturated.

The hydrolysis can also be conducted to stereo-specifically resolve racemic mixtures of optically active compounds. One group of chiral ester substrates which can be resolved in accordance with the invention can be represented by the formula:

```
              R1  O
              |   ||
I        R--C--C--OR2
              |
              X
```

wherein R and $R_1$ can be hydrogen and $C_1$-$C_{18}$

$R_2$ can be $C_1$-$C_4$ alkyl; and

X can be halogen (fluorine, chlorine, bromine or iodine), aliphatic or aromatic groups and substituted derivatives thereof wherein R, $R_1$, and X are different. Preferably, R and $R_2$ are independently $C_1$-$C_2$ and more preferably $C_1$. $R_1$ is preferably hydrogen. X is preferably halogen and more preferably halogen of chlorine or bromine.

The aliphatic or aromatic groups and substitute derivatives thereof can include phenyl, hydroxyphenyl, phenoxy, hydrophenoxy, methyl, ethyl, chloromethyl, hydroxyethyl and the like.

Illustrative of the chiral compounds which can be hydrolyzed are:

methyl-2-chloropropionate
methyl-2-bromopropionate
ethyl-2-chloropropionate
ethyl-2-bromopropionate
butyl-2-chlorobutylate
methyl-2-phenoxypropionate

The brominated compounds are less soluble than the corresponding chlorinated compounds. While compounds such as methyl 2-(p-chlorophenoxy) propionate are sufficiently insoluble in water to be enzymatically hydrolyzable without further treatment, other substituted compounds such as methyl 2-(p-hydroxyphenoxy) propionate and methyl 2-(p-hydroxyphenyl) propionate may be sufficiently water soluble to require water-insolubilization for conducting the hydrolysis.

The organic substrate phase which is used under conditions such that it is non-miscible with the aqueous phase can be comprised of a water-immiscible substrate alone or a substrate in a water-immiscible inert organic material which is a solvent for the substrate. The water-immiscible substrate phase also includes a substrate which has been made water immiscible such as by an organic solvent by the use of high salt concentration or by the use of low temperatures. Included within the term "water-immiscible" as used herein are compounds which are or can be made water immiscible.

By "water-immiscible" is meant that the ester is soluble to no more than about 50 millimolar in water at 25°C. The esters are preferably water immiscible or capable of being made substantially water immiscible without changing the chemical composition of the esters. The esters must be soluble in an organic phase which is water immiscible or capable of being insolubilized in water, i.e., soluble in water to less than 50 millimolar.

The insolubilization can be achieved by the use of an organic solvent for the ester which solvent is water immiscible, by the use of salts and buffers or any other means of insolubilizing the ester relative to the aqueous component during the enzymatic hydrolysis. Insolubilization can also be affected or improved by temperature manipulation.

By " water-immiscible solvent" it is intended to mean that the organic solvent is miscible in the water up to no more than about 10%, preferably no more than 1%, under the conditions of reaction. The organic solvent can be any water-immiscible organic solvent which is non-reactive with the substrate, reaction products, the enzyme or have substantially no inhibitory effect on the enzyme activity under the conditions of the reaction. The solvent can be heavier or lighter than water. The preferred solvent is the one most suitable for the substrate being hydrolyzed and the enzyme. This solvent can be easily determined by one of ordinary skill in the art. For example, carbon tetrachloride is a preferred solvent for such substrates as methyl 2-halopropionates. The organic solvents can be illustrated by toluene, perchloroethylene chloroform, methylene chloride, carbon tetrachloride, cyclohexanone, and the like. One or ordinary skill in the art can easily determine if a particular solvent adversely affects a specific enzyme or substrate. The solvent to water volume ratio can range from about 5:1 to about 1:10 with a ratio of from about 2:1 to about 1:2 being preferred. The substrate content of the organic phase at the beginning of the reaction can range from about 100% to about 5%, with from about 100% to about 25% on a weight per volume basis being preferred.

Solvents such as carbon tetrachloride, perchloroethylene and chloroform can be used in the present invention even though such solvents can inhibit enzyme activity. By binding the lipase enzyme to the substrate at the interface when dispersed, the effect of the solvent on the enzyme activity is reduced or eliminated. While carbon tetrachloride and perchloroethylene can be used effectively without restriction, chloroform, when used in large amounts, may tend to cause enzyme inactivation. The amount of solvent which can be used can be easily determined by one of ordinary skill in the art. Ester solvents which can be hydrolyzed by the enzyme or water-soluble alcohol solvents which can inactivate the enzyme are to be avoided.

The esters can also be temporarily insolubilized in aqueous solution by means of a quantity of salt sufficient to reduce the solubility of the substrate in the water to less than 50 millimolar. Representative examples of effective salts include the halides, carbonates, bicarbonates, phosphates, sulfates, nitrates and mixtures thereof of alkali metal and alkaline earth metals. The salt must be at least partially water soluble. Suitable salts are illustrated by sodium chloride, potassium chloride, sodium bicarbonate, sodium sulfate and the like.

The enzymes which can be utilized in the invention are those enzymes which can catalyze the hydrolysis of the substrate, and if stereospecificity is desired, are capable of resolving the substrate by stereospecific hydrolysis. Illustrative sources of these lipase enzymes include the genera Rhizopus, Mucor, Aspergillus, Candida, Pseudomonas, Alcaligenes, Achromobacter and Bacillus. Specific lipase enzymes include those from various non-specific species of Mucor, Pseudomonas, Achromobacter, Alcaligenes, and Rhizopus as well as those from Aspergillus niger , Candida cylindracea, Candida paralipolytica , Rhizopus japonicus, Rhizopus niveus Rhizopus delemar, Bacillus subtillus and animal digestive lipases such as porcine pancreas, and the like and mixtures thereof.

The lipase for use in resolving the chiral esters of the invention is preferably obtained from the yeast Candida cylindracea . This enzyme has been found to produce the highest yield of resolved isomers in the process of the invention and is considered the most desirable for a commercial process.

The type of lipase enzyme can vary depending on the substrate. It is is desired to resolve a racemate, the enzyme must be stereoselective for one of the isomers of any particular substrate. The enzymes can be used as a crude preparation or in pure form though the crude preparation is preferable for economic reasons. Enzymes stabilized through chemical modifications which retain their solubility in water can also take advantage of the special properties of the process of the invention.

The lipase enzymes, due to their molecular structure, adhere to the substrate at the interface. For best results, not more than about 2d% of the total enzyme and preferably not more than 5% of the total enzyme is present in the aqueous phase at equilibrium.

The enzyme/substrate are reacted under conditions conducive to that enzyme. For enzymes such as Candida cylindracea lipase, temperatures within the range of from about 0°C. to about 45°C. and pH's within the range of from about 3 to about 8 are preferred. Enzyme amounts can be easily determined by one of ordinary skill in the art.

The reaction can be conducted in any convenient vessel equipped with an agitation means sufficient to form the required dispersion. Means are provided for continuously washing the reaction mixture at an approximately constant flow to remove enzyme inhibiting or inactivating substances. Inasmuch as the enzymatic reaction is pH sensitive and

an acid is produced during the reaction, appropriate neutralizing agents can be added to effect the washing. The reaction vessel is preferably equipped with a pH monitor, such as a pH probe to initiate addition of the neutralizing agent based on the data from the pH probe. Preferably, the reaction vessel is equipped with a pH activated controller unit which optionally controls the pH of the reaction by automatically feeding a neutralizing agent to the solution. Such neutralizing agents include, but are not limited to, aqueous solutions of hydroxides, carbonates and bicarbonates of the alkali and alkaline earth metals as well as ammonium hydroxide, though sodium bicarbonate and ammonium hydroxide are the preferred neutralizing agents.

A critical aspect of the process of the present invention is removing the alcohol and other water-soluble inhibitory products from the reaction mixture while retaining the enzyme in the reactor. Since the alcohol is water soluble, it is necessary to separate the aqueous phase from the water-immiscible organic phase. In order to limit removal of the enzyme from the reaction mixture during separation of the aqueous phase, the aqueous solution must be separated from the water-immiscible organic phase under dispersion conditions such that the enzyme is tied up at the interface between the two phases. A convenient method of accomplishing this end is by the use of a large diameter settling tube or separation tube in the reactor which allows the dispersion to be isolated in a quiescent state in the reactor. In the quiescent state, the aqueous phase will separate from the organic phase. The enzyme will remain attached to the dispersed organic phase and the alcohol will be found in the aqueous phase. The aqueous phase can be removed along with portions of the alcohol while not removing portions of the enzyme. If the solvent is heavier than water, the separation tube will allow removal of the aqueous phase from above. A solvent lighter than water could be used if the aqueous phase is removed from the bottom.

The process of the invention can be operated such that the enzyme inhibitory substances can be washed out of the reaction system without washing out substantial amounts of enzyme while maintaining the volume of the reaction mixture at a low level. In order to accomplish this, amounts of the aqueous phase containing the inhibitory substances are removed in amounts substantially equal to the amount of wash water or neutralizing agent added. Preferably, the amount of reaction mixture does not vary by more than about ± 20% over the initial reaction mixture amount. Simultaneously operating pumps which add and remove equal quantities can be used. In large systems external separation can be used where the organic phase is recycled to the reactor. Portions of the reaction mixture can be pumped out of the reactor and into a separation device such as a low-speed centrifuge which separates the aqueous phase from the organic phase. The organic phase, along with a sufficient part of the aqueous phase or water to maintain the level in the reactor constant, can then be recycled.

A portion of the hydrolysis product, i.e., the acid, if water soluble, can also be recovered continuously from the aqueous phase. If the isomeric unreacted ester is the desired product, this can be recovered by final centrifugation of the rection mixture at the end of the desired extent of reaction. If an organic solvent is used, the unreacted ester product is then recovered by distillation. If the isomeric unreacted ester is the desired product, the isomeric acid formed can be racemized, reesterified and returned for resolution by techniques well known to one of ordinary skill in the art.

The process of the invention allows for the conduct of a continuous enzymatic reaction while washing out enzyme inhibitors or reaction products generated during the reaction without a substantial loss in the enzyme. Water-soluble enzyme can be used in a continuous reaction without the need to immobilize the enzyme by conventional techniques.

The present invention will be more fully illustrated in the examples that follow:

## EXAMPLE 1

Three reactions of 100 milliliters each were carried out separately to compare the process of the invention with prior art processes. Reaction (a) was carried out in accordance with the invention. Reactions (b) and (c) were carried out as batch reactions.

(a) 50 milliliters of DL-methyl-2-bromopropionate (DL-MBP), 50 milliliters of water, 0.1 grams of $CaCl_2$, 2.5 grams of $NaHCO_3$, and 100 milligrams of a lipase enzyme from Candida cylindracea having an activity of about 2300 international units (2 milligrams of lipase per milliliter of DL-MBP) were mixed in a reaction vessel of the type shown in Figure 1. The reaction vessel was equipped with a pH monitor and a pH activated controller unit, an inlet tube for the addition of a 5% solution of $NaHCO_3$ to the reaction vessel, a phase separation outlet tube of large diameter and a magnetic stirrer. The outlet tube was positioned below the liquid level of the reaction mixture and allowed phase separation to take place within the tube. The substrate DL-MBP is heavier than water so that the aqueous phase was separated as the upper layer in the tube. Both the inlet and the outlet tubes were connected to equal flow pumps

which operated simultaneously. When the pH in the reactor dropped below 6.2, $NaHCO_3$ solution was automatically added and an equal volume of the separated aqueous phase in the phase separation tube was withdrawn.

After 24 hours of reaction, under vigorous agitation and at a temperature of about 22°C, the reactor was dismantled to recover the product. The reaction yielded 15 milliliters of L-(-)-MBP and 96 milliliters of aqueous solution. The optical rotation of the product was -68.7. This result corresponds to an extent of reaction of about 70% and an optical purity of the product of about 94%.

In addition, 378 milliliters of aqueous solution was collected through the outlet pump. This 378 milliliters contained about 35 milligrams or about 35% of the original quantity of enzyme added to the reaction. For a stirred (completely backmixed) reactor where enzyme is present in aqueous solution under continuous washing, one would expect by calculation that 98% of the enzyme would be washed out of the reactor over the period of the reaction. Thus, the loss of only 35% of the enzyme over the period of the reaction indicates that the enzyme which is water soluble and should be removed with the aqueous fraction is immobilized in the reaction system. The ability of the enzyme to be attracted to the substrate at the interface between the substrate and the aqueous phase allows the aqueous phase to be separated therefrom without depleting the enzyme as long as the substrate-aqueous phase interface is maintained.

(b) A batch reaction was conducted using 50 milliliters DL-MBP, 50 milliliters of water, 0.1 grams $CaCl_2$, 23.38 grams $NaHCO_3$, and 100 milligrams of lipase enzyme from Candida cylindracea having an activity of 2300 international units (2 milligrams of lipase per milliliter of DL-MBP). The reactants were mixed together and agitated continuously at room temperature. The reaction started at pH 8.0 and rapidly dropped to 7.2.

The reaction vessel was tested for the extent of hydrolysis and product purity after 24 hours by removing 10 milliliters of the reaction mixture and centrifuging to separate the D-MBP product phase from the reaction mixture. Approximately 3.5 milliliters of L-MBP was separated from the 10 milliliters of reaction mixture. The optical rotation of the L-MBP product was -28.37. This corresponds to an extent of reaction of 30% and product optical purity of 68%.

After an additional 72 hours of reaction time, the reaction was again evaluated by removing a 25 milliliter sample and analyzing it as before. The extent of reaction was found to be 44% and the optical purity was 73%.

This reaction thus failed to produce 90% or better purity product even after 96 hours of reaction.

(c) 20 milliliters of DL-methyl-2-bromopropionate, 80 milliliters water, 0.1 gram $CaCl_2$, 9.35 grams $NaHCO_3$ and 40 milligrams Candida cylindracea lipase enzyme as identified hereinbefore (2 milligrams of lipase per milliliter of DL-MBP) were mixed and agitated continuously at room temperature. The initial pH of the reaction mixture was 8.0 which dropped rapidly to about 7.2. Thereafter the pH dropped gradually to about 5.5 as the reaction progressed. After 24 hours, the reaction mixture was tested for extent of hydrolysis and product purity. The extent of reaction was 67% and optical purity of the product (L-(-)-MBP) was 93%. This reaction produced 6.6 milliliters final product in 100 milliliters of reaction mixture.

The process of the present invention provided 15 milliliters of product in a reaction medium of approximately 100-110 milliliters constant volume. The extent of reaction after 24 hours was 70% at 94% optical purity. In batch reaction (b), using the same volume and the same amount of substrate as in batch a) with a ratio of water to substrate of 1:1, after 24 hours the extent of reaction was only 30% at an optical purity of about 68%, and after 96 hours the extent of reaction was only 44% at an optical purity of 73%. In batch reaction (c), using a ratio of water to substrate of 4:1 (prior art accepted process), after 24 hours the extent of reaction was about 67% at an optical purity of about 93%. However, this reaction provided only 6.6 milliliters product per 100 milliliters reaction mixture whereas the invention provided 15 milliliters of product in a final reaction mixture volume of 111 milliliters. This series of examples shows the requirement for using 4:1 water to substrate ratio if a high extent of reaction and optical purity are to be obtained in a batch reaction and that at a 1:1 ratio poor results are obtained. The present invention obtains a high extent of reaction, good optical purity and good yield in a lower reactor volume.

## EXAMPLE 2

50 milliliters of DL-methyl-2-chloropropionate (DL-MCP) were dissolved in 50 milliliters of carbon tetrachloride (4.4 molar MCP in $CCl_4$). This 100 milliliters of organic phase was mixed with 100 milliliters water, 0.1 gram $CaCl_2$, 5 grams $NaHCO_3$ and 100 milligrams Candida cylindracea lipase enzyme and allowed to react in a reactor as described in Figure 1 and Example 1 without an automatic pH controller. The temperature was controlled at 13°C. When the pH dropped from the initial pH of 8.0 to 6.2, a solution of 0.61 molar

$NH_4OH$ was pumped into the reactor. Equal quantities of aqueous phase were removed as in Example 1. Since an automatic pH controller was not used, the $NH_4OH$ solution was pumped into the reaction mixture in an amount sufficient to maintain the pH within the range of about 4.0-7.0.

After 22 hours of reaction, the addition of base was discontinued. The final pH was about 4.0. The aqueous phase was separated from the organic phase, and 63 milliliters of organic ($CCl_4$) phase was obtained. The concentration of product (L-(-)-MCP) in the organic phase was 2.53 molar. The optical rotation measured on the $CCl_4$ product solution was -6.26. From this information it was determined that the reaction had gone to 64% completion and the optical purity of the product was 84%. A total of 370 milliliters of $NH_4OH$ solution was pumped through the reactor.

EXAMPLE 3

50 milliliters of DL-methyl-2-bromopropionate were placed in a round bottomed flask and dispersed in 50 milliliters of 5% sodium bicarbonate containing 0.2% calcium chloride and 100 milligrams of a lipase from Candida cylindracea having an activity of 2300 international units. The flask was equipped as in Example 1(a).

As the enzyme reaction proceeded at 19°C, the original pH of 8 dropped to 6.2, whereupon automatic pH control at pH 6.2 using 5% sodium bicarbonate solution was initiated. Volumes of aqueous phase equal to the neutralizer added were withdrawn through the outlet tube. After an approximately 11-hour reaction period, 425 milliliters of 5% sodium bicarbonate solution had been added. At the termination of the reaction, 378 milliliters of aqueous phase had been removed, 96 milliliters of aqueous phase and 14.5 milliliters of organic phase remained in the reactor. On the basis of milliliters charged, the yield was 29% at 94% purity of L-(-)-methyl-2-bromopropionate.

The total lipase in 378 milliliters of the collected aqueous phase was assayed to be 34.65 milligrams or 34.65%. According to sodium bicarbonate consumption, the reaction went to 63.4%.

EXAMPLE 4

70 milliliters of DL-methyl-2-bromopropionate, 70 milliliters of 5% sodium bicarbonate solution containing 0.2% calcium chloride and 140 milligrams of lipase were reacted as in Example 1(a).

After 2 hours of reaction, the aqueous phase was selectively reduced so that the total volume of the reaction mixture was about 110 milliliters. During overnight reaction, the volume went up. The finally recovered volume was 110 milliliters of aqueous phase and 21 milliliters of L-(-)-methyl-2-bromopropionate product. The total aqueous phase collected over the 25 hour reaction period was 663 milliliters. The total volume of neutralizer used was 459 milliliters. Product yield was approximately 30% at 94% purity. According to the sodium bicarbonate used, the reaction went to 62% of completion.

The reaction can be performed equally as well using DL-methyl-2-chloropropionate dissolved in carbon tetrachloride. Formation of $CO_2$ by the sodium bicarbonate neutralization may cause volume build-up in the reactor. Ammonium hydroxide may be more preferable as a neutralizing agent to overcome this problem.

## Claims

1. A process for conducting a lipase enzymatic reaction comprising dispersing a water-immiscible organic substrate-containing phase in an aqueous phase in the presence of lipase enzyme, said dispersal being sufficient to bind substantially all of the lipase enzyme at the interface between the aqueous phase and the organic phase, allowing the lipase enzyme to react with the substrate while so bound, washing the dispersed phase with an aqueous solution and separating portions of the aqueous phase from the organic phase during said reaction.

2. The process of Claim 1 wherein said lipase enzyme is Candida cylindracea lipase.

3. The process of Claim 1 wherein the enzymatic reaction is a stereoselective resolution of a racemate.

4. The process as recited in Claim 3 wherein the enzyme is a stereoselective lipase for the substrate.

5. The process as recited in Claim 1 wherein said substrate-containing phase contains a water-immiscible solvent which is a solvent for the substrate.

6. The process as recited in Claim 5 wherein said solvent is carbon tetrachloride.

7. The process as recited in Claim 1 wherein said enzymatic reaction generates an acid and the acid is neutralized by a neutralizing agent as the reaction proceeds.

8. The process as recited in Claim 7 wherein said acid is neutralized continuously as it is generated.

9. The process as recited in Claim 7 wherein a portion of the aqueous phase substantially equal to the added neutralizing agent is removed as the neutralizing agent is added.

10. The process of Claim 1 wherein the ratio of the aqueous phase to the substrate phase ranges from about 5:1 to about 1:10.

11. The process of Claim 1 wherein the aqueous phase is separated from the substrate phase external to the reaction.

12. A process for stereoselectively resolving in a continuous reaction racemic mixtures of optically active chiral ester substrates defined by the formula

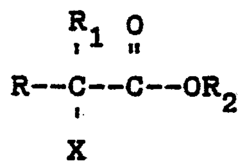

wherein R and $R_1$ represents hydrogen and $C_1$-$C_{18}$ alkyl; $R_2$ represents $C_1$-$C_4$ alkyl and X represents halogen, aliphatic or aromatic groups and substituted derivatives thereof, wherein R, $R_1$ and X are different in a continuous reaction comprising:

(a) dispersing a substrate-containing phase in an aqueous phase in the presence of a lipase enzyme capable of stereoselectively hydrolyzing said substrate, said dispersal being sufficient to bind substantially all of the enzyme to the substrate at the interface between the aqueous phase and the substrate phase;

(b) allowing the enzyme to react with the substrate while so bound; and

(c) continuously neutralizing with a neutralizing agent the dispersed phases while removing an amount of the aqueous phase substantially equivalent to the amount of neutralizing agent added.

13. The process of Claim 12 wherein the ratio of the aqueous phase to the substrate phase ranges from about 5:1 to about 1:10.

14. The process of Claim 12 wherein the aqueous phase is separated from the substrate phase external to the reaction.

15. The process as recited in Claim 12 wherein the substrate is an alkyl ester of 2-halopropionic acid.

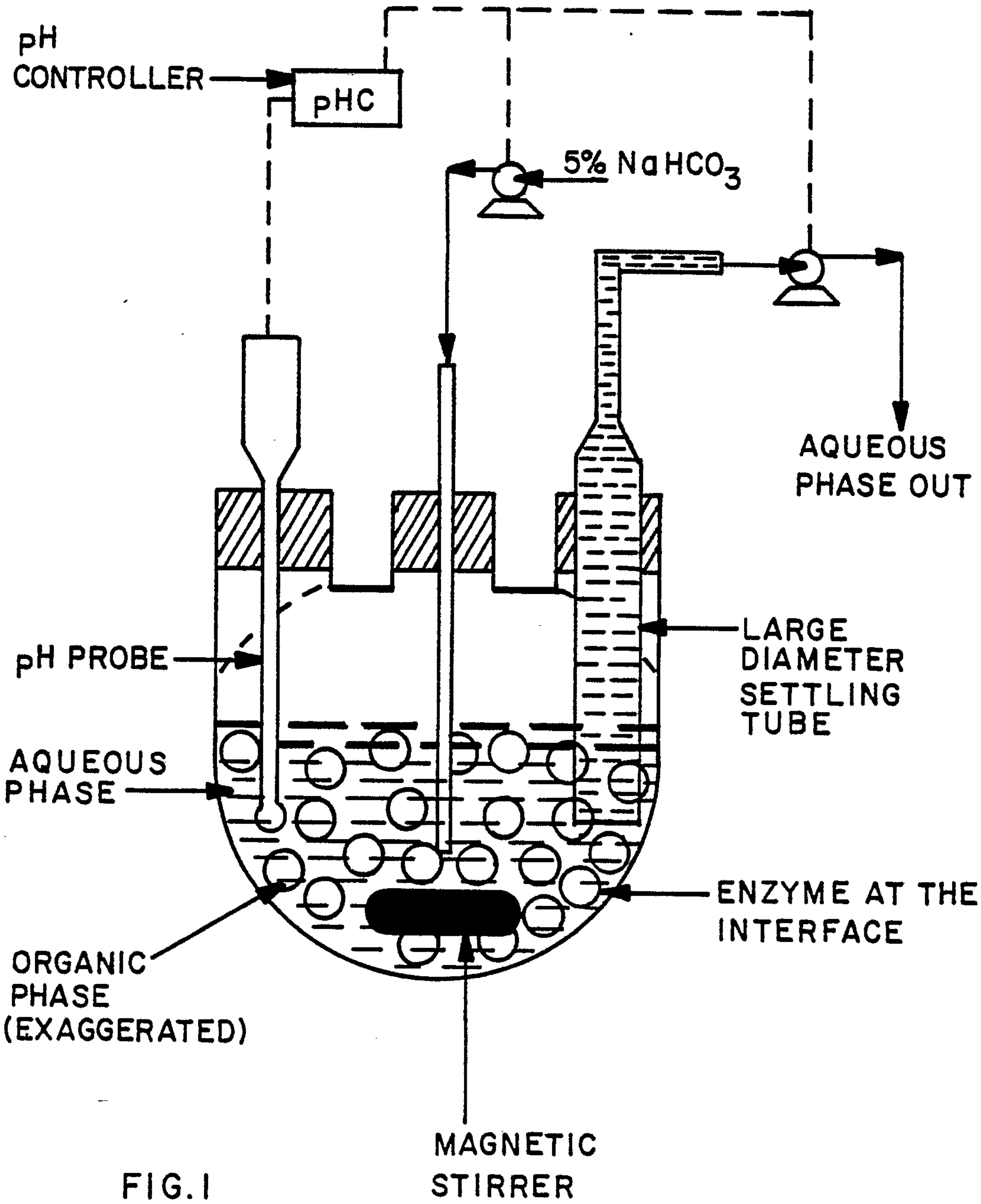
pH
CONTROLLER
pHC
5% $NaHCO_3$
AQUEOUS
PHASE OUT
LARGE
DIAMETER
SETTLING
TUBE
pH PROBE
AQUEOUS
PHASE
ENZYME AT THE
INTERFACE
ORGANIC
PHASE
(EXAGGERATED)
MAGNETIC
STIRRER

FIG.1